# NEW EUROPEAN PATENT SPECIFICATION

(11) **EP 1 272 197 B2**
(45) Date of publication and mention of the opposition decision: **28.03.2012**
(45) Mention of the grant of the patent: 15.11.2006
(21) Application number: 01914069.8
(22) Date of filing: 01.03.2001
(51) Int. Cl.: A61K 31/66, A61K 31/375, A61P 25/16, A61P 1/16, A61P 7/02, A61P 31/00, A61P 3/10

(54) **COMBINATION OF LECITHIN WITH ASCORBIC ACID**
KOMBINATION DES LEZITHINS MIT ASCORBINSÄURE
COMBINAISON DE LECITHINE AVEC L'ACIDE ASCORBIQUE

(30) Priority: 06.03.2000 HU 0000997 P
(43) Date of publication of application: 08.01.2003
(73) Proprietor: N.V. Nutricia, 2712 HM Zoetermeer (NL)
(72) Inventor: JAVOR, András, H-1118 Budapest (HU); PELLIONISZNE PAROCZAI, Margit, H-1125 Budapest (HU); SZEKELY, Akosné, H-1204 Budapest (HU)
(74) Representative: van Westenbrugge, Andries
(86) International application number: PCT/HU2001/000027
(87) International publication number: WO 2001/070206

(56) References cited:
- EP-A- 0 326 829
- WO-A-93/19624
- WO-A-95/26646
- WO-A-99/43220
- WO-A-99/43329
- CN-A- 1 096 421
- CZ-B- 281 571
- GB-A- 2 280 110
- JP-A- 02 265 457
- JP-A- 10 094 382
- KR-B- 148 462
- KR-B- 178 559
- US-A- 4 780 456
- US-A- 5 077 069
- US-B1- 6 180 139
- KOUMANS A K ET AL: "Nutrition and atherosclerosis: some neglected aspects." CLINICAL CARDIOLOGY, (1985 OCT) 8 (10) 547-51. , XP001052686

## Description

The present invention relates to the use of a combination of lecithin and ascorbic acid for the preservation or increase of memory and physical performance of sick people.

In the commercial and legal practice the name of lecithin means the mixture of polar and non-polar lipids from vegetable or animal source, containing acetone insoluble components minimum in 60%. About 60% of acetone insoluble part of lecithin is the mixture of phosphor containing lipids, which has as a main component L-alpha-phosphatidylcholine (Drug and Cosmetic Industry, February, 1992.). According to scientific terminology the name of lecithin means only L-alpha-phosphatidylcholine. In the description of the invention the name of lecithin is used in commercial and legal meaning.

In living body inside it in vertebrates and so in human, too, phospholipids and their decomposing products have vital importance role. In human body among phospholipids L-alpha-phosphatidylcholine can be observed in the largest volume, which is the most significant phospholipid from the point of pharmacological and biological view. L-alpha-phosphatidylcholine can be found mainly in membranes of cells, where it is present as a structure component and as an initial compound of different biological active agents. This double role of L-alpha-phosphatidylcholine is essential for the sake of the integrity and functional action of cells (Lecithin and health care, Semmelweis Verlag, Hoya, Germany, 1985; Nutrition Review, Vol. 52, 327-339, 1994.)

It is also important to optimal cell function the chemical structure and ratio of membrane components (proteins, lipids). If the favourable ratio of components changes, for example on the effect of outside factors (malnutrition or unhealthy food intake, physical trauma, bacterial or virus infection) or if the chemical structure of membrane components changes, for example because of peroxidation, they may induce depending on the type of cell and tissue different illnesses, for example vascular wall damage (atherosclerosis).

During the biological ageing the ratio of components of cell membranes and so their structures also change because the increase of cholesterol and decrease of L-alpha-phosphatidylcholine content, which result the decrease of cell function and increase of vulnerability of cells.

One possibility to assure the optimal composition of cell membranes as well as to protect their chemical structure mainly against with peroxidation is that if the main component of membrane phospholipids is present in enough amount and simultaneously membranes are protected from peroxidation with a natural antioxidant such as ascorbic acid.

Lecithin from animal or vegetable source because its complex physiological role has been used by medical science for a long time as a general roborant, at healthy and sick people. Recently, lecithin for this purpose is used alone (Buerlecithin) or in combination with vitamins and trace elements (Gerovit capsule, Kondi tablet Hungarian products). Lecithin, beside its general tonic action is also used for therapeutic aim in some illnesses, for example in ageing memory disturbance caused by acetylcholine deficit (senile dementia) or in moving inco-ordination (tardive dyskinesia). In these cases lecithin promotes as a favourable choline source to the synthesis of acetylcholine. Probably this property of lecithin explains its favourable adjuvant effect in Alzheimer disease (Biological Psychiatry, Vol. 17, 275-280, 1982), where mainly the destruction of cholinerg neurons (neurons which synthesise acetylcholine) can be observed in part because the increased production of free radicals. Another therapeutic benefit of lecithin is that it decreases the high lipid and cholesterol level of blood attenuating the risk of atherosclerosis (Lecithin and health care, Semmelweis Verlag, Hoya, Germany, 1985; Clinical Cardiology Vol. 8, 547-551, 1985). For the increase of the antiartherosclerotic effect of lecithin it can mix with natural oils having high polyenoic long chain fatty acids (US patent, No. 4 780 456). Recently, the hepatoprotective effect of lecithin was discovered, which is also used for therapeutic aim (Nutrition Review, Vol. 52, 327-339, 1994.).

In human beings the role of ascorbic acid otherwise vitamin-C was seen from its discovery (1928) to several decades in that it needs to the synthesis of collagen to prevention of scurvy. However, in recently more and more biological functions of ascorbic acid have become known mainly from that time when its antioxidant property and the presence of free radicals in the living body were discovered.

Free radicals are chemically very active molecules because they have redundant electron. These molecules for example superoxide-anion (O₂⁻), hydroxyl free radical (OH⁻) and free radical generating hydrogen-peroxide (H₂O₂) are formed during the normal metabolic processes of cells and can react with macromolecules (proteins, lipids and deoxyribonucleic acid) changing hereby their structures. These changes may endanger the normal function of cell in great degree. For the sake of prevention of danger effect of free radicals cells have defensive mechanisms and protective agents such as ascorbic acid, which can adsorb and neutralise free electrons. The preventive effect of ascorbic acid is very important in stress situation, when the production of free radicals is augmented significantly because the increased cell function. During the stress large volume of ascorbic acid is secreted in part with preventive aim from tissue stores to blood carrying it to different cells. However, if the degree of stress is higher than the possibilities of preventive mechanisms, for example stress is very high or chronic or the amount of ascorbic acid or other antioxidants are not enough, cells and tissues will be damage, which will manifest in the form of different diseases such as atherosclerosis, decreased immune system activity, decreased viability of some neurons, Parkinson disease.

Ascorbic acid beside its free radical scavenger activity decreases high serum cholesterol saving hereby the arteries from atherosclerosis (Clinical Cardiology Vol. 8, 547-551, 1985), and affects significantly the function of neurones, too, for example increases the secretion of acetylcholine and noradrenaline neurotransmitters as shown by in vitro neuronal study, and its large doses (1-3 g/day) stimulate the effect of antipsychotic drugs in schizophrenic people (Progress in Neurobiology Vol. 43, 537-565, 1994).

Because ascorbic acid is essential for human organism and has positive effect in several diseases it is used alone and in combination with other active ingredients, for example with antioxidants (carotenoids (GB patent No. 2 280 110), vitamin E), with antipyretics (acetylsalicilic acid, paracetamol) and with metal ions (calcium, magnesium and iron).

On the basis of biological knowledge of lecithin (L-alpha-phosphatidylcholine) and ascorbic acid it may be presumable that using them in combination they can complete each other's effects. However when we studied lecithin ascorbic acid combination in animal experiments we experienced surprising manner that mixing the two compounds in an adequate ratio and giving it to rodents the effect of blend was more potent than the effect of components separately or the sum of the separated effect of components. This type of drug interaction is said to potentiating synergism, which was appeared in the increase of learning and physical performances of animals. The present invention therefore provides the use of lecithin with 10.00-99.99 mass% L-alpha-phosphatidylcholine content and ascorbic acid in 3:1 mass ratio beside the known substances for manufacture of a food product, a refreshing beverage or a concentrate for their production for the improvement of the physical and cognitive performance of sick people.

Studying the effect of combination and its components separately on the learning process of rats in water labyrinth (Brain Research Bulletin Vol. 45, 475-488, 1998) it was found that, rats treating with combination could find out from labyrinth with fewer error and in the first and third session in shorter time than vehicle (control) or only lecithin or only ascorbic acid treated animals (Table I.). Ascorbic acid and lecithin were ineffective when they were given separately (Table I.).

**Table I. Effect of ascorbic acid (Vit.-C), lecithin and their combination on the learning process of normal rats in water labyrinth**

| | Number of session | Control | Vit.-C | Lecithin | Lecithin+Vit.-C |
|---|---|---|---|---|---|
| Dose (mg/kg/day) p.o. | | | 2×10 | 2×30 | 2×(30+10) |
| Number of animals | | 10 | 10 | 10 | 10 |
| Number of errors Mean±S.E.M. | 1 | 16.0±0.39 | 16.4±0.45 | 15.8±0.29 | 13.2±0.46**‡ |
| | 2 | 10.9±0.77 | 11.6±0.40 | 11.8±0.57 | 8.7±0.97† |
| | 3 | 5.4±0.52 | 6.2±0.76 | 6.1±0.62 | 3.2±0.55*‡ |
| | 4 | 2.9±0.72 | 3.9±0.35 | 3.2±0.59 | 2.6±0.67 |
| Swimming time (sec) Mean±S.E.M. | 1 | 138.4±8.95 | 140.3±9.54 | 129.5±7.61 | 110.7±8.87^{#a} |
| | 2 | 90.7±7.80 | 89.9±6.22 | 80.1±5.54 | 75.9±8.59 |
| | 3 | 51.6±8.17 | 64.9±7.29 | 62.9±6.34 | 46.1±5.38^{ab} |
| | 4 | 29.9±2.33 | 34.8±2.79 | 40.1±3.66^{#} | 42.3±7.57 |

| | | | | | |
|---|---|---|---|---|---|
| * p<0.05, ** p<0.01 compared with control by Mann-Whitney test ^{#} p<0.05 compared with control by Student's t-test † p<0.05 ‡ p<0.01 compared with Vit.-C and lecithin by Mann-Whitney test ^{a} p<0.05 compared with Vit.-C by Student's t-test ^{b} p<0.05 compared with lecithin by Student's t-test | | | | | |

The composition of lecithin: 78.4% phospholipid (inside it 63.1% L-alpha-phosphatidylcholine, 24.1% L-alpha-phoshatidylethanolamine, 7.6% L-alpha-phosphatidilinositol, 4.6 % L-alpha-phoshatidic acid) and 20 % other lipid.

In water labyrinth learning test the combination prevented the increase of error number and swimming time caused by scopolamine a memory-destroying agent (Table II.). The two measured parameters were almost normalised by lecithin ascorbic acid blend (Table II.)

Studying the effect of combination and its components separately on the physical performance of mice in swimming test (Pharmacological Research, Vol. 23, 149-155, 1991) it was found that mice treating with the combination could swim longer time than vehicle (control) or only lecithin or only ascorbic acid treated animals (Table III.). In the study ascorbic acid was ineffective, when it was given in itself (Table III.).

**Table II. Effect of lecithin ascorbic acid (Vit.-C) combination on the learning process of rats in scopolamine induced memory deficit in water labyrinth**

| | Number of session | Placebo | Scopolamine | Scopolamine |
|---|---|---|---|---|
| Dose (mg/kg/day) p.o. | | control | control | Lecithin+Vit.-C 2×(30+10) |
| Number of animals | | 10 | 10 | 10 |
| Number of errors Mean±S.E.M. | 1 | 14.0±0.68 | 16.2±0.83 | 13.9±0.72 |
| | 2 | 6.6±0.76 | 11.6±0.52⁺⁺ | 8.8±0.59** |
| | 3 | 2.8±0.53 | 8.9±0.41⁺⁺ | 5.1±0.50** |
| | 4 | 2.0±0.33 | 6.3±0.26⁺⁺ | 3.9±0.48** |
| Swimming time (sec) Mean±S.E.M. | 1 | 96.4±8.44 | 114.7±9.45 | 78.1±5.31^{##} |
| | 2 | 57.5±5.69 | 73.7±6.22 | 59.7±3.94 |
| | 3 | 37.6±4.08 | 66.7±6.47^{nm} | 41.7±3.21^{##} |
| | 4 | 27.1±1.36 | 42.4±2.78^{nm} | 40.5±3.96 |

| | | | | |
|---|---|---|---|---|
| *p<0.05, **p<0.01 compared with scopolamine control by Mann-Whitney test ^{##}p<0.01 compared with scopolamine control by Student's t-test ⁺⁺p<0.01 compared with normal control by Mann-Whitney test ^{nm}p<0.01 compared with normal control by Student's t-test | | | | |

**Table III. Effect of ascorbic acid (Vit.-C), lecithin and their combination on the swimming time of normal mice**

| | | | Number of days | | | | |
|---|---|---|---|---|---|---|---|
| | Dose (mg/kg/day) p.o. | Number of animals | 1. day starting values | 2. day | 3. day | 4. day | 5. day |
| | | | Swimming time (sec) mean ± S.E.M. | | | | |
| Control | | 10 | 77.4±4.5 | 80.4±3.9 | 82.2±5.9 | 84.6±7.3 | 84.0±6.8 |
| Vit.-C | 2×10 | 10 | 76.2±3.7 | 70.8±3.8 | 82.2±3.4 | 88.8±6.1 | 90.0±6.9 |
| Lecithin | 2×30 | 10 | 70.2±3.6 | 100.2 ±6.9* | 120.6±7.8** | 126.0±8.0** | 126.6 ±9.1** |
| Lecithin+Vit-C | 2×(30+10) | 10 | 72.0± 3.2 | 106.8 ± 11.9* | 132.6 ± 9.4** | 144.0 ± 13.9** | 141.0 ± 13.0** |

| | | | | | | | |
|---|---|---|---|---|---|---|---|
| * p<0.05, ** p<0.01 compared with control by Student's t-test | | | | | | | |

On the basis of the results of these experiments it can be established that lecithin is mixed with ascorbic acid in an appropriate ratio and using this blend in suitable formula we can gain such a drug product, which can be applied more favourable and more effectively to those areas where these agents are used alone or maybe together but no optimised ratio. In addition, in the combination the smaller dose of lecithin and ascorbic acid can produce as effect as ingredients separately decreasing by this means the side effects of ingredients. The higher dose of ascorbic acid may promote kidney stone white the high dose of lecithin, higher than 25 g/day may produce loss of appetite, nausea, stomach puffing and diarrhoea.

In the combination the mass ratio of lecithin and ascorbic acid is 3:1, while the daily doses of components depending on the aim of application can vary between 1.5-500 mg/kg in the case of lecithin and between 1-60 mg/kg in the case of ascorbic acid dividing these doses into suitable portion.

In a preferable drug formula lecithin is present in well dispersed solid or emulsified or liposome or dissolved form, which contain ascorbic acid solid or dissolved form. The blend of two ingredients can be used in oral and parenteral drug formula according to need together with auxiliary materials, which are used for drug formulation. In these drug forms the mass of two ingredients can vary as a dose between 1-1000 mg respectively.

In what follows mentioned according to Example 1 produced capsule can be favourable used for prevention of illnesses. In illnesses as an additive treatment for tonic aim 2×2, 3×2 or 4×2 capsule and for the sake of tendentious effect, for example for the prevention of ageing memory disorders or Alzheimer disease or Parkinson diseases 3×2, 4×2 or 4×3 capsule may be favourable.

The following non-limiting examples further illustrate the invention:

### Example 1

### Formulation of lecithin and ascorbic acid combination in 3:1 ratio in hard gelatine capsule

2 part by mass lecithin is suspended into 4.5 part by mass sunflower oil at 45-50 °C. Sunflower oil has to contain as an antioxidant. 0.065 part by mass vitamin E oil. After the suspending of lecithin the oil is cooled on 20 °C and 0.66 part by mass well pulverised (micronized) ascorbic acid is suspended into it. Finally this oil is formulated in 0.7 ml hard gelatine capsules.

### Example 2 (NOT PART OF INVENTION)

### Formulation of lecithin and ascorbic acid combination in 20:1 ratio in hard gelatine capsule

2 part by mass lecithin is suspended into 4.5 part by mass sunflower oil at 45-50 °C. Sunflower oil has to contain as an antioxidant 0.065 part by mass vitamin E oil. After the suspending of lecithin the oil is cooled on 20 °C and 0.1 part by mass well pulverised (micronized) ascorbic acid is suspended into it. Finally this oil is formulated in 0.7 ml hard gelatine capsules.

### Example 3 (NOT PART OF INVENTION)

### Formulation of lecithin and ascorbic acid combination in oil injection

Among sterile circumstance using sterile ingredients without pyrogen the injection is made according to the example 1 and the oil suspension of the combination is filled into 2 ml ampoule. This injection can be applied subcutan or intramuscular route.

### Example 4

### Formulation of lecithin and ascorbic acid combination in oil/water type injection

Among sterile circumstances using sterile ingredients without pyrogen 3 part by mass lecithin is suspended into 4.5 part by mass sunflower oil at 45-50 °C. Sunflower oil has to contain as an antioxidant 0.075 part by mass vitamin E oil. After the suspending of lecithin the oil is cooled on 20 °C, and it is emulsified in distilled water, which contains 0.66 part by mass ascorbic acid. The water phase is 1.5 times of oil phase. In this emulsion the emulsifier is itself lecithin. The emulsion is filled into 2 ml ampoule.

## Claims

1. Use of lecithin with 10.00 - 99.99 mass% L-alpha-phosphatidylcholine content and ascorbic acid in 3:1 mass ratio beside the known substances for manufacture of a food product or a refreshing beverage or a concentrate for their production for the improvement of the physical and cognitive performance of sick people.

## Patentansprüche

1. Verwendung von Lezithin mit einem Anteil von 10,00 - 99,99 Massen% L-Alpha-Phosphatidylcholin und Ascorbinsäure in einem 3:1-Massenverhältnis neben den bekannten Stoffen zur Herstellung eines Nahrungsmittelprodukts oder eines Erfrischungsgetränks oder eines Konzentrats zu ihrer Herstellung zur Verbesserung der physischen und kognitiven Leistungsfähigkeit von kranken Menschen.

## Revendications

1. Utilisation d'une lécithine ayant une teneur en L-alpha-phosphatidylcholine de 10,00 - 99,99 % en masse et d'acide ascorbique dans un rapport en masse de 3:1 outre les substances connues pour la fabrication d'un produit alimentaire ou d'une boisson rafraîchissante ou d'un concentré pour leur production pour l'amélioration des performances physiques et cognitives de personnes malades.
